# EUROPÄISCHE PATENTSCHRIFT

(11) **EP 2 194 866 B1**
(45) Veröffentlichungstag und Bekanntmachung des Hinweises auf die Patenterteilung: **27.06.2012**
(21) Anmeldenummer: 08783471.9
(22) Anmeldetag: 05.09.2008
(51) Int. Cl.: A61B 5/08, A61K 49/00

(54) **VORRICHTUNG, GASMISCHUNG UND VERFAHREN ZUR LUNGENDIAGNOSTIK**
DEVICE, GAS MIXTURE AND METHOD FOR LUNG DIAGNOSIS
DISPOSITIF, MELANGE DE GAZ ET PROCEDE DE DIAGNOSTIC PULMONAIRE

(30) Priorität: 07.09.2007 CH 14072007
(43) Veröffentlichungstag der Anmeldung: 16.06.2010
(73) Patentinhaber: Eco Medics AG, 8635 Dürnten (CH)
(72) Erfinder: ISLER, Rudolf, CH-8132 Hinteregg (CH)
(74) Vertreter: Ritscher, Thomas
(86) Internationale Anmeldenummer: PCT/CH2008/000371
(87) Internationale Veröffentlichungsnummer: WO 2009/030058

(56) Entgegenhaltungen:
- EP-A- 1 764 035
- WO-A-02/24070
- DE-A1- 4 318 690
- US-A1- 2007 191 726

## Beschreibung

Die Erfindung betrifft eine lungendiagnostische Vorrichtung, eine dafür geeignete Testgasmischung sowie ein Verfahren zur Bestimmung der Ventilationshomogenität (VH) an einem spontan atmenden oder beatmeten Patienten.

Zur Messung von Parametern der Lungenfunktion werden unter anderem Methoden angewendet, die ein- und ausgeatmete Volumina möglichst genau und ohne Beschwerden des Patienten messen können. Bei bekannten Systemen werden die Atemvolumina meist mit Sensoren bestimmt, welche die mittlere Flussgeschwindigkeit (Flow) in einer bekannten Querschnittsfläche des Sensors bestimmen.

Zu den hierfür verwendeten bekannten Vorrichtungen gehören insbesondere die Differentialdruckmessgeräte, z.B. Pneumotachographen, Turbinen, Thermistoren, Vortex-Flowmeter und das Ultraschall-Laufzeitverfahren.

Die genannten Vorrichtungen haben verschiedene Nachteile, die ihre Anwendungsmöglichkeiten beschränken, insbesondere einen zu begrenzten linearen Messbereich, eine Abhängigkeit von der Gaszusammensetzung und -temperatur, einen zu hohen Messwiderstand, ein ungünstiges Frequenzverhalten, eine problematische Sterilisierbarkeit sowie eine zu aufwendige und/oder zu oft nötige Kalibrierung. Auch ist die meist erforderliche Kooperation des Patienten nicht immer gesichert.

Im Zusammenhang mit Untersuchungen von Atmungsstörungen bei cystischer Fibrose (CF) ist die Verwendung von Gasgemischen mit Anteilen an inerten, aber spezifisch relativ schweren Gasen, wie Schwefelhexafluorid, zur Bestimmung der Ventilationsverteilung in der Lunge vorgeschlagen worden (Van Mullen, A. et Baren, D., Pediatric Pulmonology 30:30-9 (2000)), und zwar in der Erwartung, dass sich unterschiedliche Verteilungen der unterschiedlich schweren Gasanteile verstärkt bei pathologischen Lungen (COPD, CF) verändern. Ferner ist es bekannt, dass die Molekularmassensumme (MM) der Inspirations- und Exspirationsgasmischung mittels Ultraschalbnessung direkt im Atemstrom gemessen werden kann (Buess, Ch. et al, IEE Trans. Biomed. Eng., 33(8) 768 -774, 1986)), wie auch in EP-A-1 764 035 gezeigt.

Aufgabe der Erfindung ist eine verbesserte Bestimmung der Ventilationshomogenität (VH) an spontan atmenden oder beatmeten Patienten in einer messtechnisch einfachen Weise.

Diese Aufgabe wird gemäss einer ersten Ausführungsform der Erfindung gelöst durch eine Vorrichtung mit den Merkmalen von Anspruch 1, d.h. eine Vorrichtung zur Bestimmung der Ventilationshomogenität an einem spontan atmenden oder beatmeten Patienten mit wenigstens einer Quelle für ein atembares Testgasgemisch (auch vereinfach als Testgas bezeichnet), das im Wesentlichen die gleiche Molekularmassensumme wie Umgebungsluft oder ein Vergleichsgasgemisch hat, sich aber von beiden durch Zusatz wenigstens eines spezifisch schwereren Inertgases und gegebenenfalls wenigstens eines spezifisch leichteren Inertgases zur Kompensation der Molekularmassensumme unterscheidet, und einer Einrichtung zur Bestimmung der Molekularmassensummen des vom Patenten exhalierten Testgasgemischs.

Das gegebenenfalls an Stelle von Umgebungsluft verwendete Vergleichsgasgemisch oder Vergleichsgas ist ein atembares und pharmakologisch unbedenkliches Gas oder Gasgemisch mit einem ausreichenden Sauerstoffgehalt zwischen dem von Umgebungsluft und 100 Vol.%, gegebenenfalls mit einen Zusatz eines spezifisch leichteren Gases wie Helium, um die Molekularmassensumme des Vergleichsgases der von Umgebungsluft anzunähern oder anzugleichen.

Bevorzugte Ausführungsformen der erfindungsgemässen Vorrichtung haben die in den Ansprüchen 2 - 9 angegebenen Merkmale.

Gemäss einer zweiten Ausführungsform bietet die Erfindung ein Testgasgemisch mit den in Anspruch 10 angegebenen Merkmalen, d.h. ein Testgasgemisch zur Bestimmung der Ventilationshomogenität an einem spontan atmenden oder beatmeten Patienten, nämlich ein atembares Gasgemisch, das im Wesentlichen die gleiche Molekularmassensumme wie die Normalluft hat, sich aber von dieser durch einen Zusatz wenigstens eines spezifisch schwereren Inertgases und gegebenenfalls (d.h. wenn bzw. da das Vergleichsgasgemisch bzw. die Umgebungsluft bereits ein spezifisch leichteres Inertgas in mehr oder weniger grossen Anteilen enthält) eines spezifisch leichteren Inertgases unterscheidet.

Bevorzugte Ausführungsformen des Testgases haben die in den Ansprüchen 11 - 14 angegebenen Merkmale.

Gemäss einer weiteren Ausführungsform bietet die Erfindung ein Verfahren mit den in Anspruch 15 angegebenen Merkmalen, d.h. Verfahren zur Bestimmung der Ventilationshomogenität an einem spontan atmenden oder beatmeten Patienten, wobei der Patient aktiv oder passiv und kontrolliert ein atembares Testgasgemisch inhaliert, das im Wesentlichen die gleiche Molekularmassensumme wie Umgebungsluft oder ein Vergleichsgasgemisch hat, sich aber von beiden durch einen Zusatz wenigstens eines spezifisch schwereren Inertgases und gegebenenfalls wenigstens eines spezifisch leichteren Inertgases unterscheidet und die Molekularmassensumme (MM) des vom Patienten exhalierten Testgases bestimmt wird, um aus der Differenzen zwischen den Molekularmassensummen (MM) des vom Patienten inhalierten und exhalierten Testgasgemisches die Ventilationshomogenität des Patienten zu beurteilen.

Das erfindungsgemässe Testgasgemisch hat trotz seines Gehaltes an spezifisch relativ schweren Anteilen insgesamt im Wesentlichen die gleiche Molekularmassensumme, wie normale Atemluft oder ein Vergleichsgasgemisches die gleiche Atembarkeit wie normale Umgebungsluft, weil der Anteil des spezifisch schweren Gases durch den Anteil des spezifisch leichteren Gases ausgeglichen ist.

Die spezifisch leichteren Anteile des Testgasgemisches verteilen sich in der Lunge verhältnismässig schneller als die spezifisch schwereren Anteile. Auf diese Weise lassen sich die unterschiedlichen Zeitkonstanten bei pathologischen Lungenbefunden (z.B. COPD, CF) besser, zuverlässiger und einfacher ermitteln, weil sich die relativen kurzzeitigen Veränderungen der Zusammensetzung der ausgeatmeten Luft besonders dann mit bekannten Verfahren, wie der Ultraschallmessung, feststellen lassen, wenn das Testgasgemisch, das die spezifisch schwerere Komponente enthält, im Wesentlichen die gleiche Molekularmassensumme besitzt, wie die Umgebungsluft bzw. das Vergleichsgasgemisch ohne eine spezifisch schwerere Komponente.

Ein atembares Gasgemisch ist allgemein ein solches, bei dem Sauerstoff in einem ausreichenden Anteil von vorzugsweise mindestens etwa 21% oder mehr enthalten ist und das pharmakologisch unbedenklich und ansonsten gegenüber den Lungen im Wesentlichen inert ist.

Wenn anstelle von Umgebungsluft als Vergleichsgasgemisch eine atembare Gasmischung ohne die spezifisch schwerere Komponente verwendet wird, kann dieses Gemisch Sauerstoff in einer Konzentration von 21 bis 100, vorzugsweise 30 bis 100, mehr bevorzugt 50 bis 100, am meisten bevorzugt 100 Vol.% und gegebenenfalls eine spezifisch leichtere Komponente, wie Helium zur Kompensation der durch einen vergrösserten Sauerstoffgehalt erhöhten Molekularmassensumme enthalten. Im allgemeinen wird ein Vergleichsgasgemisch anstelle von Umgebungsluft verwendet, wenn ein erhöhter Sauerstoffgehalt bei der Beatmung medizinisch ratsam oder notwendig ist.

Vorzugsweise unterscheidet sich die Molekularmassensumme des Testgasgemisches und des Vergleichsgasgemisches bzw. des Vergleichsgases um weniger als 10 %, vorzugsweise um weniger als 5 %, mehr bevorzugt um weniger als 2 %, am meisten bevorzugt um weniger als 1 % von der Molekularmassensumme der Umgebungsluft Vergleichsgasgemisches.

Sowohl das Testgasgemisch als auch die gegebenenfalls anstelle von Umgebungsluft verwendet Vergleichsgas bzw. Vergelichsgasgemisch hat vorzugsweise eine Molekularmassensumme von 28 bis 33 g/mol, wobei etwa 29 oder 32, insbesondere 28,85 oder 32,0 g/mol (z.B. im Fall von Normalluft aus 100 % Sauerstoff) bevorzugt sind.

In einer Ausführungsform ist das wenigstens eine spezifisch schwerere Inertgas (I2) in dem Testgasgemisch aus der Gruppe gewählt, die Argon, Neon, Krypton, Radon, Xenon und SF₆ sowie Mischungen derselben umfasst, wobei Radon und SF₆ bevorzugt sind und SF₆ aus messtechnischen Gründen besonders bevorzugt ist.

Das wenigstens eine spezifisch leichtere Inertgas im Testgasgemisch oder gegebenenfalls dem Vergleichsgasgemisch ist Helium.

Zur Bestimmung der Molekularmassensumme (MM) des vom Patienten (P) exhalierten Testgasgemisches wird vorzugsweise ein Gerät zur Messung der Ultraschallgeschwindigkeit in Gasen oder Gasgemischen und eine Einrichtung zur Umrechnung der gemessenen Ultraschallgeschwindigkeiten in die Molekularmassensumme und eine Einrichtung zur Anzeige der gemessenen Werte verwendet.

Die erfindungsgemässe Vorrichtung kann eine zweite Quelle für das Vergleichsgasgemisch umfassen, das vorzugsweise einen höheren Sauerstoffanteil als Umgebungsluft hat, keinen Zusatz des spezifisch schwereren Gases und gegebenenfalls zur Kompensation eines erhöhten Sauerstoffanteils einen Zusatz an spezifisch leichterem Inertgas, wie Helium, enthält.

Gewünschtenfalls besitzt die Einrichtung zur Bestimmung der Molekularmassensumme eine Einrichtung zur Umrechnung der gemessenen Werte auf Standardbedingungen (z.B. Normierung).

Zweckmässigerweise ist die erfindungsgemässe Vorrichtung so ausgebildet, dass zwischen der Quelle für das Testgasgemisch und einer Quelle für das Vergleichsgasgemisch oder auf Umgebungsluft umgeschaltet werden kann.

Das erfindungsgemäss durchführbare Diagnoseverfahren beruht ganz allgemein auf der Messung der Molekularmassensumme (MM) von Gasmischungen und einem Unterschied zwischen der Molekularmassensumme der inhalierten und der exhalierten Gasmischung. Hintergrund ist die unterschiedliche Diffusionsgeschwindigkeit der beiden Gasanteile, welche auf Grund der unterschiedlichen Stoßquerschnitte und auf Grund der unterschiedlichen molaren Massen die mittlere freie Weglänge verändert. Die MM der Umgebungsluft entspricht in etwa 28,85 g/mol (+ 0,1 g/mol je nach Temperatur und Feuchte). Es ist für Fachleute einsichtig, dass auch andere von der MM direkt abgeleitete mathematische Derivate, z.B. MM im Verhältnis zum Atemvolumen, CO₂-Gehalt, etc., vorzugsweise vom Begriff MM als solches mit umfasst werden.

Die erforderlichen Messungen lassen sich mit für andere Zwecke bekannten Mitteln problemlos durchführen. Geeignete Messeinrichtungen zur Durchführung der Erfindung sind z.B. Geräten zur Messung der Ausbreitungsgeschwindigkeit von Schall- bzw. Ultraschallwellen im fliessenden Medium, Geräte zur Bestimmung der Wärmeleitzähigkeit oder der Licht- bzw. UV- oder IR-Adsorption sowie elektroakustischen Messgeräte.

Eine erfindungsgemäss besonders bevorzugte Vorrichtung zur Bestimmung der Molekularmassensumme des verwendeten Gasgemischs beruht auf der Messung von absoluten Schallaufzeiten im Haupt- und/oder Nebenstrom, insbesondere Ultraschall(US)-Laufzeiten, zur Messung der momentanen Atemstromgeschwindigkeit und damit auch der Atemstromvolumina. Dies ermöglicht eine hohe Linearität, eine Unabhängigkeit von der Gaszusammensetzung und Temperatur, eine hohe zeitliche Auflösung sowie sehr gute Hygieneeigenschaften.

Einrichtungen für die Ultraschall-Laufzeitanalyse bieten ausserdem die Möglichkeit einer gleichzeitigen Bestimmung der Molmasse fliessender Gase bzw. der mittleren Molmasse fliessender Gasgemische sowie Informationen über Gasfluss und Gaszusammensetzung in Echtzeit.

Die hier zur Bezeichnungen der Gaskomponenten verwendeten Bezeichnungen "spezifisch schwerer" bzw. "spezifisch leichter" beziehen sich dabei auf das spezifische Gewicht der Hauptkomponenten der Atemluft, Stickstoff und Sauerstoff. Da Stickstoff in einem atembaren Gasgemisch durch andere Inertgase ersetzt werden kann, Sauerstoff hingegen für die Atembarkeit kritisch ist, werden die eben genannten Bezeichnungen daher auf Sauerstoff bezogen.

Erfindungsgemässe Testgasgemische und Vergleichsgasgemische sind homogene Gemische mit einer definierten Zusammensetzung, deren Genauigkeit im Allgemeinen besser als 0.1% (+/- 0.02 g/mol) betragen soll. Die für medizinische Zwecke erforderliche Reinheit (z.B. p.a.) muss natürlich gewährleistet sein.

Die Bezeichnung "Molekularmassensumme" (MM) bezieht sich auf die Molekularmasse, die sich aus den relativen Anteilen der Komponenten und ihren jeweiligen Molekularmassen ergibt. Die Molekularmassensumme einer Mischung aus 50% Sauerstoff (Molekularmasse 32) und 50% Helium (Molekularmasse 4) ergibt sich beispielsweise zu 36/2 = 18.

Die Bezeichnungen "Vergleichsgasgemisch" oder "Vergleichsgas" beziehen sich auf ein atembares Gas, das die gleiche Molekularmassensumme wie das Testgasgemisch hat, aber keine spezifisch schwerere Komponente enthält und die spezifisch leichtere Komponente dann enthält, wenn das Vergleichsgasgemisch, wie meist bevorzugt, die gleiche Molekularmassensumme wie Umgebungsluft haben soll.

Die Bezeichnung "im Wesentlichen", "ungefähr" oder "etwa" vor einer Zahlenangabe bedeutet hier eine Abweichung von ± 10%, vorzugsweise ± 5 %, mehr bevorzugt ± 2 %, vorzugsweise weniger als ±1 %. Angaben in Prozent beziehen sich hier, sofern nicht anders vermerkt, im Zusammenhang mit der Angabe von Gasgemischen auf das Volumen.

Das Betreiben der Vorrichtung bzw. das Verfahren der Erfindung erfordert keine Kooperation des Patienten wie bei anderen herkömmlichen Lungenfunkdonsmessungen. Vorrichtung und Verfahren sind daher besonders gut für Komapatienten und Neugeborene geeignet. Beispielhafte und bevorzugte Anwendungen von Verfahren und Vorrichtungen gemäss der Erfindung liegen zusätzlich zu den oben genannten allgemeinen und speziellen Indikationen insbesondere auch in folgenden Bereichen:
Objektivierung von Ventilationsstörungen (obstruktiv, restriktiv, kombiniert);
Prüfen der Reversibilität von Verteilungsstörungen (Bronchospasmolysetest);
Provokation von Ventilationsstörungen (unspezifischer oder spezifischer Provokationsstest);
Verlaufsparameter von COPD (chronic obstructive pulmonary disease),
Früherkennung von CF (cystische Fibrose; als Verlaufsparameter);
Evaluation von Medikamentwirkungen auf Verteilungsstörungen.

Im Folgenden wird Luft (Tabelle 1,Gas A) tabellarisch einem erfindungsgemässen Testgasgemisch (Tabelle 2, Gas B) mit im Wesentlichen gleicher MM als eine bevorzugte Ausführungsform gegenüber gestellt.

**Tabelle 1.**

| **Luft Gas A** | **Atommasse** | **% Gas** | **MM** |
|---|---|---|---|
| O | 15.9994 | 21.00 | 0.00 |
| O₂ | 31.9988 | 21.00 | 6.72 |
| He | 4 | 0.00 | 0.00 |
| N | 14.0067 | | 0.00 |
| N₂ | 28.0134 | 79.00 | 22.13 |
| S | 32.064 | | 0.00 |
| F | 18.9984 | | 0.00 |
| SF₉ | 146.0544 | 0.00 | 0.00 |
| Total | | 100.00 | **28.85** |

**Tabelle 2**

| **Testgas Gas B** | **Atommasse** | **% Gas** | **MM** |
|---|---|---|---|
| O | 15.9994 | | 0.00 |
| O₂ | 31.9988 | 21 | 6.72 |
| He | 4 | 29.5 | 1.18 |
| N | 14.0067 | | 0.00 |
| N₂ | 28.0134 | 43.5 | 12.19 |
| S | 32.064 | | 0.00 |
| F | 18.9984 | | 0.00 |
| SFe | 146.0544 | 6 | 8.76 |
| Total | | 100 | **28.85** |

Im Folgenden wird ein Vergleichsgas (Gas C) aus im Wesentlichen reinem Sauerstoff zusammen mit einem erfindungsgemässen Testgas (Gas D) gleicher MM als weiteres bevorzugtes Beispiel einer Testgas/Vergleichgas-Paarung gezeigt.

**Tabelle 3**

| **Vergleichsgas Gas C** | **Atommasse** | **% Gas** | **MM** |
|---|---|---|---|
| O | 15.9994 | | 0.00 |
| O₂ | 31.9988 | 100.00 | 32.00 |
| He | 4 | 0.00 | 0.00 |
| S | 32.064 | | 0.00 |
| F | 18.9984 | | 0.00 |
| SF₆ | 146.0544 | 0.00 | 0.00 |
| Total | | 100.00 | **32.00** |

**Tabelle 4**

| **Testgas Gas D** | **Atommasse** | **% Gas** | **MM** |
|---|---|---|---|
| O | 15.9994 | | 0.00 |
| O₂ | 31.9988 | 65.00 | 20.80 |
| He | 4 | 28.10 | 1.12 |
| S | 32.064 | | 0.00 |
| F | 18.9984 | | 0.00 |
| SF₆ | 146.0544 | 6.90 | 10.08 |
| Total | | 100.00 | **32.00** |

In der folgenden Tabelle 5 ist ein erfmdungsgemäss bevorzugtes Testgasgemisch aus Helium, Radon und O₂ mit im Wesentlichen gleicher MM wie Umgebungsluft angegeben.

**Tabelle 5**

| **Komponenten** | **Atommasse** | **% Anteil** | **MM** |
|---|---|---|---|
| **O₂** | 31.9988 | 21 | 6.72 |
| **He** | 4 | 26.7 | 1.07 |
| **CO₂** | 35.9988 | 0 | 0.00 |
| **N2** | 28.0134 | 49 | 13.73 |
| **S** | 32.064 | | 0.00 |
| **F** | 18.9984 | | 0.00 |
| **SF₆** | 146.0544 | 0 | 0.00 |
| **Neon** | 20.17 | 0 | 0.00 |
| **Argon** | 39.94 | | 0.00 |
| **Krypton** | 83.8 | | 0.00 |
| **Xenon** | 131.2 | 0 | 0.00 |
| **Radon** | 222 | 3.3 | 7.33 |
| **Total** | | 100 | **28.84** |

Es ist für Fachleute einsichtig, dass die Kenntnis der Atemruhelage für die Interpretation der Messresultate und deren Reproduzierbarkeit wesentlich ist. Die Atemgase CO₂ und O₂, und/oder auch die Atemminutenvolumina liefern wesentliche Informationen über die Atemruhelage und können für die Interpretation der Messdaten als Referenzpunkte benutzt werden.

Um Patienten dabei zu unterstützen, in ihrer optimalen Atemruhelage zu atmen, ist ein "Incentive-Screen" oder "Animations- Vorrichtung" mit oder ohne direktem "Biofeedback" Display eine weitere interessante Anwendung der vorliegenden Erfindung.

Die Erfindung wird nun in vier Figuren sowie anhand von nicht beschränkenden Beispielen weiter erläutert. In den Figuren zeigen:
Fig. 1 das Schema einer erfindungsgemässen Vorrichtung.
Fig. 2 drei zusammen dargestellte Messkurven von zweimal drei Atemzügen mit unterschiedlichen Messparamtern auf der Ordinate und der Zeit auf der Abszisse;
Fig. 3 die Messkurven eines Patienten mit normaler Ventilationshomogenität, wobei Molekularmassensumme in Prozent der Minimal- und Ma ximalwerte auf der Ordinate und das Atemvolumen in Litern auf der Ab szisse aufgetragen ist;
Fig. 4 die analogen Messkurven wie in Fig. 3, jedoch für einen COPD-Patienten mit gestörter Ventilationshomogenität;.
Fig. 5 zeigt die Veränderung der Molekularmassensummen der ersten Exspiration von Vergleichsgas nach komplettem Einwaschungsvorgang von Testgas.

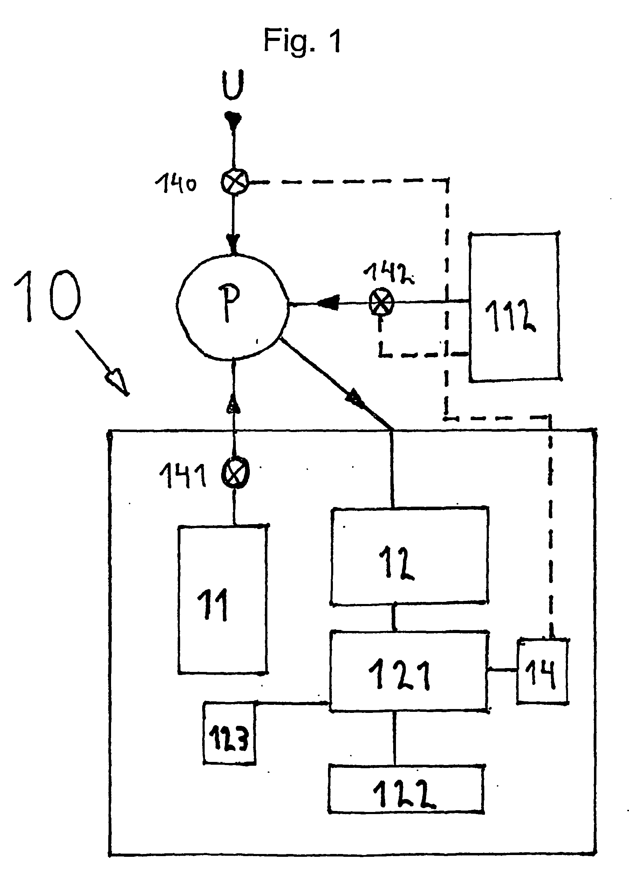

Die schematisch in Fig. 1 dargestellte Vorrichtung 10 besitzt wenigstens eine Quelle 11 für ein atembares Gasgemisch der Erfindung, das praktisch die gleiche Molekularmassesumme wie die Umgebungsluft hat, sich von dieser aber durch einen Zusatz wenigstens eines spezifisch leichteren Inertgases, wie Helium, und wenigstens eines spezifisch schwereren Inertgases, wie Schwefelhexafluorid, unterscheidet, und eine Einrichtung 12 zu Bestimmung der Molekularmassensumme des vom Patienten exhalierten Gasgemisches der Erfindung und der exhalierten Umgebungsluft.

Die Vorrichtung 10 kann zusätzlich zur Quelle 11 für das atembare Gasgemisch der Erfindung ein Vergleichsgasgemisch aus einer zweiten Quelle 112 aufweisen. Dieses Gasgemisch tritt an die Stelle der Umgebungsluft (U), wenn ein höherer Sauerstoffgehalt bei der Beatmung erforderlich ist. Da die Molekularmassensumme MM dieses Vergleichsgasgemisches mit erhöhtem Sauerstoffanteil vorzugsweise gleich der MM der Umgebungsluft und des Testgasgemisches der Erfindung ist und der erhöhte Sauerstoffanteil zweckmässig durch einen verminderten Stickstoffgehalt kompensiert wird, kann der Einfluss des erhöhten Anteils des spezifisch schwereren (als Stickstoff) Sauerstoffs durch Zusatz eines spezifisch leichteren Inertgases, insbesondere Helium, kompensiert werden. Dies erübrigt sich, wenn das Testgasgemisch die gleiche Molekularmassensumme hat wie das Vergleichsgas.

Die Einrichtung 12 zur Bestimmung der Molekularmassensumme des vom Patienten (P) exhalierten Gasgemisches der Erfindung ist vorzugsweise ein Gerät zur Messung der Ultraschallgeschwindigkeit in Gasen oder Gasgemischen. Die Einrichtung 121 zur Umrechnung der gemessenen Ultraschallgeschwindigkeiten in die Molekularmassensumme und die Einrichtung 122 zur Anzeige der gemessenen Werte können Teil der erfindungs-gemässen Vorrichtung 10 sein oder getrennt von dieser angeordnet werden. Die Vorrichtung 10 kann ferner eine an sich bekannte Einrichtung 123 zur Umrechnung der gemessenen Werte auf Standardbedingungen umfassen.

Gemäss einer bevorzugten Ausführungsform besitzt die Vorrichtung 10 ferner Einrichtungen 14; 140; 141; 142 zur Umschaltung des vom Patienten (P) inhalierten Gasgemisches von Umgebungsluft (U) auf das aus der Quelle 11 abgegebene Gasgemisch der Erfindung und umgekehrt, bzw. zur Umschaltung des vom Patienten (P) inhalierten zweiten Gasgemisches aus der Quelle 112 auf das Gasgemisch der Erfindung aus der Quelle 11 und umgekehrt.

In der Praxis wird z.B. ein für andere Zwecke kommerziell erhältliches Gerät verwendet, wie es von der Anmelderin unter den Marken Exhalyzer D® bzw. Spiroson Scientific® erhältlich ist und zwei Ultraschall (US)-Sender/Empfangereinheiten besitzt, die seitlich an einem die Luftströmung leitenden Atemrohr angebracht sind. Zur Bestimmung der Flussgeschwindigkeit werden die folgenden Schritte durchgeführt: In einem ersten Messzyklus wird ein US-Puls von einem der US-Sender/Empfänger ausgestrahlt, durchläuft den Messkanal, und damit die Atemströmung, und wird schliesslich vom gegenüberliegenden US-Sender/Empfänger empfangen. Die Laufzeit des Schallimpulses wird mittels digitaler Elektronik präzise bestimmt (Auflösung 10 ns).

In einem darauf folgenden Messzyklus wird ein US-Puls in umgekehrter Richtung ausgesendet und dessen Laufzeit bestimmt. Da sich der Schallimpuls auf seinem Pfad einmal gegen und einmal mit der Gasströmung fortpflanzt, unterscheiden sich die beiden Laufzeiten.

Vorzugsweise werden für die praktische Durchführung der Erfindung US-Sender/Empfänger eingesetzt, wie sie aus der Mikrofontechnik bekannt sind und das Aussenden und präzise Empfangen von sehr kurzen Schallimpulsen ermöglichen. Dies ermöglicht eine optimale Schallübertragung und die Messung der absoluten Schall-Laufzeiten. Zur Berechnung können bekannte Mittel der Digitalelektronik eingesetzt werden. Insgesamt lassen sich durch Optimierung der Sende/Empfangs-Elektronik Messungen der US-Laufzeiten mit einer Auflösung im Bereich von 10 ns erreichen.

Vorzugsweise wird die Messung in Leit-Rohren mit kleinen Rohrdurchmessern von typisch im Bereich von 30 bis 6 mm durchgeführt, wobei die mechanische Ausgestaltung des Messrohres natürlich auch den Anforderungen einer einwandfreien Hygiene entsprechen soll.

### Beispiel 1

Gas 1 (Atmosphärenluft) und Gas 2 (Gasgemisch der Erfindung) haben eine identische Molekularmassensumme von 28.9 g/mol.

Der Sauerstoffgehalt beider Gase beträgt wenigstens etwa 21%. Die dem Gasgemisch der Erfindung zugemischten Gase (He/SF₆) sind inert, d.h. sie werden vom Körper nicht aufgenommen. Anstelle der normalen atmosphärischen Luft kann ein weiteres Gasgemisch mit erhöhtem Sauerstoffanteil verwendet werden, wenn aus medizinischen Gründen mit erhöhtem Sauerstoffgehalt gearbeitet werden muss.

Die Zusammensetzung der Gase 1 und 2 ist in der nachfolgenden Tabelle 6 angegeben.

**Tabelle 6**

| | Gas 1 | MM | Gas 2 | MM |
|---|---|---|---|---|
| | Anteil % | (g/mol) | Anteil % | (g/mol) |
| O₂ | 21 | 6.72 | 21 | 6.719748 |
| N₂ | 79 | 22.130586 | 32 | 8.964288 |
| He | | 0 | 39 | 1.561014 |
| SF₆ | | 0 | 8 | 11.684352 |
| Total | 100 | 28.9 | 100 | 28.9 |

Bei der Messung wird Atemzug pro Atemzug nun die Differenz der MM (dMM) zwischen der Inspiration und der Exspiration gemessen. Schaltet man von Gas 1 (Vergleichgas oder Umgebungsluft) auf Gas 2 (Testgas), so werden bei einer homogen ventilierten Lunge die leichten und die schweren Komponenten der Gase in einem für eine Lunge mit homogener Ventilation in einem für solche Lungen typischen Verhältnis abgeatmet. Die MM-Normkurve ist gut reproduzierbar.

Bei einer nicht homogen ventilierten Lunge ist das Verhältnis des Exspirations-Gasgemisches stärker unterschiedlich, weil sich das leichte Helium (He) im Residualvolumen (das Luftvolumen in der Lunge, das auch nach einer forcierten Ausatmung noch in der Lunge verbleibt) schneller verteilt als das schwere SF₆. Der Unterschied zwischen dieser MM-Auswaschkurve zur MM-Normkurve kann gewünschten Falls rechnerisch ausgewertet werden.

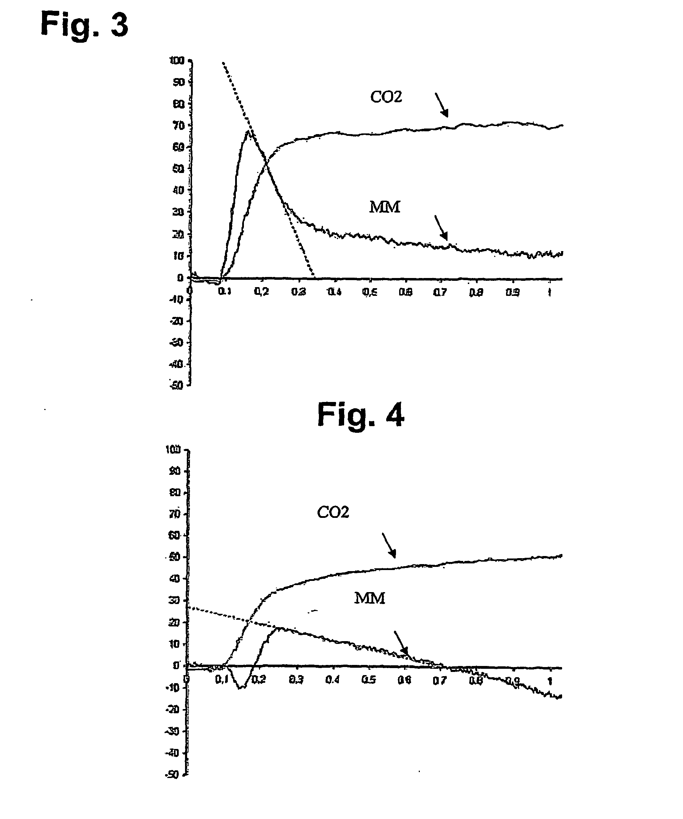

Beispielsweise verbleiben beim ersten Atemzug von Gas 2 ca. 50 % He-Anteil und 25 % SF₆ Anteil in der Lunge, d.h. es werden 50% He-Anteil und 75 % SF6 Anteil in der Exspiration des ersten Atemzuges gemessen und es ist ein starker MM-Anstieg (s. Fig.3, Pfeil in der unteren Kurve) im Vergleich zur CO₂-Kurve (Pfeil in der oberen Kurve) zu erkennen.

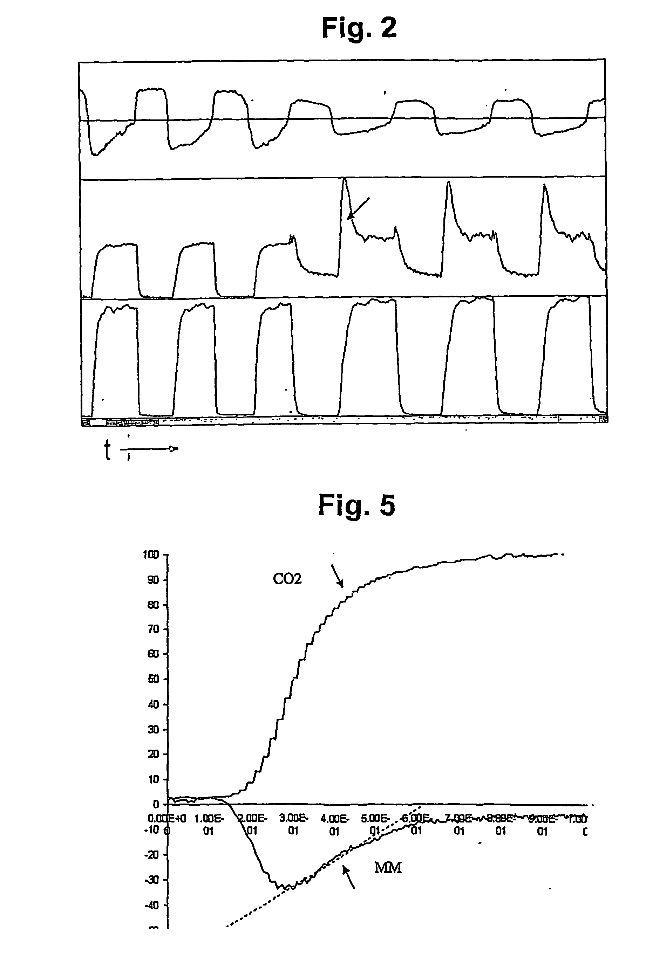

Ist auch das Residualvolumen komplett mit Gas 2 eingewaschen, so schaltet man zurück auf Gas 1 und man sieht das reziproke Phänomen - He wird schneller aus der Lunge eliminiert als SF₆ und man misst einen starken MM Abfall bei den ersten Atemzügen von Gas 1 (s. Fig. 5, Pfeil in der unteren Kurve).

### Beispiel 2

Die Figur 2 zeigt ein Beispiel einer erfindungsgemässen Messung von drei Atemzügen Gas 1 (Luft) und drei Atemzügen Gas 2. (Flow (obere Kurve; Inspiration 'plus' Exspiration 'minus'); MM, SF₆/He (mittlere Kurve), CO₂ (untere Kurve)). In der Exspiration ist das MM Signal bei Gas 1 mit dem CO₂ Signal identisch, da CO₂ schwerer als Luft ist, d.h. das MM Signal entspricht im Wesentlichen dem CO₂ Signal. Da sich nun SF₆ (hohe MM) in der Lunge schlechter als Helium verteilt (He) (tiefe MM), kommt im Verhältnis bei der ersten Exspiration mit Gas 2 mehr SF6 aus der Lunge zurück als He. Somit erkennt man einen markanten Anstieg der MM (siehe Pfeil). Dieser Anstieg nimmt kontinuierlich ab, bis die Lunge homogen eingewaschen ist.

### Beispiel 3

Die Fig. 3 und 4 sind jeweils Aufzeichnungen von MM versus exspiriertes Volumen bei einem gesunden und einem lungenkranken Menschen mit dem erfindungsgemässen Gasgemisch. Der untere Pfeil markiert die CO₂-Kurve, der untere Pfeil die MM-Kurve des ersten expirierten Atemzugs mit Gas 2. Vergleicht man eine gesunde Lunge (Fig. 3) mit einer COPD (chronic obstructive pulmonary disease)- Lunge (Fig. 4) (chronisch starker Raucher), so ist dieser Anstieg in der Exspiration der MM-Kurve des ersten exspirierten Atemzugs mit Gas 2 markant unterschiedlich. Damit wird erklärbar, dass sich die für die Erfindung wichtige unterschiedliche Verteilung der verschieden schweren Gase bei schlecht ventilierten Lungen stärker als bei gesunden Lungen zeigt.

Allgemein können für diagnostische Zwecke die Ergebnisse eines Patienten mit den entsprechenden Normwerten einer gesunden Population oder mit den errechneten theoretischen oder den zu einem früheren Zeitpunkt gemessenen Werten desselben Patienten verglichen werden.

## Patentansprüche

1. Vorrichtung (10) zur Bestimmung der Ventilationshomogenität (VH) an einem spontan atmenden oder beatmeten Patienten (P), wobei diese:
(a) wenigstens eine Quelle (11) für ein atembares Testgasgemisch, das im Wesentlichen die gleiche Molekularmassensumme (MM) wie Umgebungsluft (U) oder wie ein Vergleichsgasgemisch aus einer zweiten Quelle der Vorrichtung (112) hat, sich aber von der Umgebungsluft (U) bzw. dem Vergleichsgasgemisch aus der zweiten Quelle (112) durch einen Zusatz wenigstens eines spezifisch leichteren Inertgases (I1) und wenigstens eines spezifisch schwereren Inertgases (I2) unterscheidet, und
(b) wenigstens eine Einrichtung (12) zur Bestimmung der Molekularmassensummen (MM) der vom Patenten exhalierten Gasgemische umfasst, wobei das wenigstens eine spezifisch schwerere Inertgas (12) im Testgasgemisch aus der Gruppe ausgewählt ist, die aus Argon, Neon, Krypton, Radon, Xenon und SF₆ sowie Mischungen derselben besteht, wobei Radon und SF₆ bevorzugt sind und SF₆ besonders bevorzugt ist.

2. Vorrichtung (10) nach Anspruch 1, **dadurch gekennzeichnet, dass** sich die Molekularmassensumme des Testgasgemisches um weniger als 10 %, vorzugsweise um weniger als 5 %, mehr bevorzugt um weniger als 2 %, am meisten bevorzugt um weniger als 1 % von der Molekularmassensumme der Umgebungsluft (U) oder des Vergleichsgasgemisches aus einer zweiten Quelle (112) unterscheidet.

3. Vorrichtung (10) nach Anspruch 1 oder 2, **dadurch gekennzeichnet, dass** das Vergleichsgasgemisch aus der zweiten Quelle (112) Sauerstoff in einer Konzentration von 21 bis 100, vorzugsweise 30 bis 100, mehr bevorzugt 50 bis 100, am meisten bevorzugt 100 Vol.% enthält.

4. Vorrichtung (10) nach einem der Ansprüche 1 bis 3, **dadurch gekennzeichnet, dass** das Testgasgemisch eine Molekularmassensumme von 28 bis 33 g/mol aufweist, wobei etwa 29 oder 32, insbesondere 28,85 oder 32,0 g/mol bevorzugt sind.

5. Vorrichtung (10) nach einem der Ansprüche 1 bis 4, **dadurch gekennzeichnet, dass** das wenigstens eine spezifisch leichtere Inertgas im Testgasgemisch Helium ist.

6. Vorrichtung (10) nach einem der Ansprüche 1 bis 5, **dadurch gekennzeichnet, dass** die Einrichtung (12) zur Bestimmung der Molekularmassensumme (MM) des vom Patienten (P) exhalierten Testgasgemisches ein Gerät zur Messung der Ultraschallgeschwindigkeit in Gasen oder Gasgemischen ist und eine Einrichtung (121) zur Umrechnung der gemessenen Ultraschallgeschwindigkeiten in die Molekularmassen-summe sowie eine Einrichtung (122) zur Anzeige der gemessenen Werte aufweist.

7. Vorrichtung (10) nach einem der Ansprüche 1 bis 6, **dadurch gekennzeichnet, dass** diese Vergleichsgasgemisch aufweist, das im Wesentlichen die gleiche Molekularmassensumme wie Umgebungsluft, jedoch einen höheren Sauerstoffanteil als diese hat und keinen Zusatz des spezifisch schwereren Gases enthält.

8. Vorrichtung (10) nach einem der Ansprüche 1 bis 7, **dadurch gekennzeichnet, dass** die Einrichtung (12) zur Bestimmung der Molekularmassensumme eine Einrichtung (123) zur Umrechnung der gemessenen Werte auf Standardbedingungen umfasst.

9. Vorrichtung (10) nach einem der Ansprüche 1 bis 8, **dadurch gekennzeichnet, dass** diese die Einrichtungen (14; 140; 141; 142) zur Umschaltung des vom Patienten (P) inhalierten Gasgemisches aus Umgebungsluft (U) auf das aus der Quelle (11) abgegebene Testgasgemisch und umgekehrt, bzw. zur Umschaltung des vorn Patienten (P) inhalierten Testgasgemisches aus der Quelle (112) auf das Testgasgemisch aus der Quelle (11) und umgekehrt umfasst.

10. Testgasgemisch zur Bestimmung der Ventilationshomogenität (VH) an einem spontan atmenden oder beatmeten Patienten, **dadurch gekennzeichnet, dass** das Testgasgemisch ein atembares Gasgemisch ist, das im Wesentlichen die gleiche Molekulannassensumme (MM) wie die Umgebungsluft (U) oder ein Vergleichsgasgemisch hat, sich aber davon durch einen Zusatz wenigstens eines spezifisch schwereren Inertgases (I2) unterscheidet, wobei das wenigstens eine spezifisch schwerere Inertgas (I2) im Testgasgemisch aus der Gruppe ausgewählt ist, die aus Argon, Neon, Krypton, Radon, Xenon und SF₆ sowie Mischungen derselben besteht, wobei Radon und SF₆ bevorzugt sind und SF₆ besonders bevorzugt ist.

11. Testgasgemisch nach Anspruch 10, **dadurch gekennzeichnet, dass** sich die Molekularmassensumme des Testgasgemisches um weniger als 10 %, vorzugsweise um weniger als 5 %, mehr bevorzugt um weniger als 2 %, am meisten bevorzugt um weniger als 1 % von der Molekularmassensumme der Umgebungsluft (U) oder des Vergleichsgasgemisches unterscheidet.

12. Testgasgemisch nach Anspruch 10 oder 11, **dadurch gekennzeichnet, dass** das Testgasgemisch eine Molekularmassensumme von 28 bis 33 g/mol aufweist, wobei etwa 29 oder 32, insbesondere 28,85 oder 32,0 g/mol bevorzugt sind.

13. Testgasgemisch nach einem der Ansprüche 10 bis 12, **dadurch gekennzeichnet, dass** das wenigstens eine spezifisch leichtere Inertgas Helium ist.

14. Verwendung eines Testgasgemisches nach einem der Ansprüche 10 bis 13 in einer Vorrichtung nach einem der Ansprüche 1 bis 9.

15. Verfahren zur Bestimmung der Ventilationshomogenität (VH) an einem spontan atmenden oder beatmeten Patienten (P), **dadurch gekennzeichnet dass** der Patient:
aktiv oder passiv und kontrolliert ein atembares Testgasgemisch inhaliert, das im Wesentlichen die gleiche Molekularmassensumme (MM) wie Umgebungsluft oder ein Vergleichsgasgemisch hat, sich aber von beiden durch einen Zusatz wenigstens eines spezifisch schwereren Inertgases (I2) unterscheidet und einen ausreichenden Anteil eines spezifisch leichteren Inertgases (I1), um die Molekularmassensumme des Testgases auf den praktisch gleichen Wert der Molekularmassensumme der Umgebungsluft oder des Vergleichsgases zu bringen, und dass die Molekularmassensumme (MM) des vom Patienten exhalierten Testgases bestimmt wird, um aus der Differenzen zwischen den Molekularmassensummen (MM) des vom Patienten inhalierten und exhalierten Testgasgemisches die Ventilationshomogenität des Patienten zu beurteilen.

## Claims

1. An apparatus (10) for determining ventilation homogeneity (VH) of a spontaneously respiring or a passively respiring patient (P) comprising:
(a) at least one source (11) for a respirable test gas mixture having substantially the same sum of molecular masses (MM) as ambient air (U) or as a comparative gas mixture from a second source (112) of said apparatus but differing from said ambient air or from the comparative gas mixture from said second source (112), respectively, by an addition of at least one specifically lighter inert gas (I1) and at least one specifically heavier inert gas (I2), and
(b) at least one device (12) for determining the summary molecular masses (MM) of gas mixtures exhaled by the patient, wherein said at least one specifically heavier gas is selected from the group consisting of argon, neon, krypton, radon, xenon, and SF₆ as well as mixtures thereof, wherein radon and SF₆ are preferred, and SF₆ being particularly preferred.

2. The apparatus (10) according to claim 1, **characterized in that** said summary molecular mass of said test gas mixture differs by less than 10 %, preferably by less than 5 %, more preferred by less than 2 % and most preferred by less than 1 % from the summary molecular mass of ambient air (U) or the comparative gas mixture from said second source (112).

3. The apparatus (10) according to claims 1 or 2, **characterized in that** said comparative gas mixture from said second source (112) contains oxygen in a concentration of from 21 to 100, preferably from 30 to 100, more preferred from 50 to 100, and most preferred 100 % by volume.

4. The apparatus (10) according to any of claims 1 - 3, **characterized in that** the test gas mixture has a summary molecular mass of from 28 to 33 g/mol, wherein about 29 or 32 , notably 28.5 or 32.0 g/mol , are preferred.

5. The apparatus (10) according to any of claims 1 - 4, **characterized in that** the at least one specifically lighter inert gas having a lower specific weight in the test gas mixture is helium.

6. The apparatus (10) according to any of claims 1 - 5, **characterized in that** the device (12) for determining the sum of molecular masses (MM) of the test gas mixture exhaled by the patient (P) is an instrument for measuring ultrasound velocity of gasses or of gas mixtures, and that it comprises a device (121) for converting the ultrasound velocities measured into the sum of the molecular masses, as well as a device (122) for indicating the values measured.

7. The apparatus (10) according to any of claims 1 - 6, **characterized in that** it comprises a comparative gas mixture having essentially the same sum of molecular masses as ambient air but having a higher oxygen content than the latter, and that it contains no addition of a specifically heavier gas.

8. The apparatus (10) according to any of claims 1 - 7, **characterized in that** the device (12) for determining the sum of the molecular masses includes a means (123) for transforming the values measured for adaption to standard conditions.

9. The apparatus (10) according to any of claims 1 - 8, **characterized in that** it includes devices (14; 140; 141; 142) for changing the gas mixture inhaled by the patient (P) from ambient air (U) to the test gas mixture provided by source (11), and vice versa, and for changing the gas mixture inhaled by patient (P) provided by source (112) to the test gas mixture from source (11), and vice versa, respectively.

10. A test gas mixture for determining ventilation homogeneity (VH) of a spontaneously respiring or passively respiring patient, **characterized in that** the test gas mixture is a respirable gas mixture having essentially the same sum of molecular masses (MM) as ambient air (U) or a comparative gas mixture, but differing therefrom by addition of at least one specifically heavier inert gas (I2), wherein the at least one specifically heavier inert gas (I2) in the test gas mixture is selected from the group consisting of argon, neon, krypton, radon, xenon and SF₆, wherein radon and SF₆ are preferred and SF₆ is particularly preferred.

11. The test gas mixture according to claim 10, **characterized in that** the sum of molecular masses of the test gas mixture differs by less than 10 %, preferably by less than 5 %, more preferred by less than 2 %, and most preferred by less than 1% of the sum of molecular masses of ambient air (U) or of the comparative gas mixture.

12. The test gas mixture according to claim 11 or 12, **characterized in that** the test gas mixture has a sum of molecular masses of from 28 or 33 g/mol, wherein 29 or 32, or notably 28.85 or 32.0 g/mol are preferred.

13. The test gas mixture according to any of claims 10 to 12, **characterized in that** the at least one specifically lighter inert gas is helium.

14. Use of the test gas mixture according to any of claims 10 - 13 in an apparatus according to one of claims 1 to 9.

15. A method of determining ventilation homogeneity (VH) of a spontaneously respiring or a passively respiring patient (P), **characterized in that** the patient actively or passively and controlled inhales a respirable test gas mixture having substantially the same sum of molecular masses (MM) as ambient air or as a comparative gas mixture; both differing, however, from the test gas mixture by addition of at least one specifically heavier inert gas (I2) to the test gas mixture which contains a sufficient proportion of a specifically lighter inter gas (I1) to bring the sum of the molecular masses of the test gas to essentially the same value as the sum of molecular masses of ambient air or of the comparative gas, and that the sum of the molecular mass (MM) of the test gas exhaled by the patient is determined for evaluating ventilation homogeneity of the patient from the differences of the sums of the molecular masses of the gasses inhaled and exhaled by the patient.

## Revendications

1. Dispositif (10) pour déterminer l'homogénéité de la ventilation (VH) chez un patient (P) respirant spontanément ou ventilé, celui-ci comprenant:
(a) au moins une source (11) pour un mélange de gaz de test respirable qui présente essentiellement la même somme de masse moléculaire (MM) que l'air ambiant (U) ou qu'un mélange de gaz de comparaison provenant d'une seconde source (112) du dispositif, mais qui se distingue de l'air ambiant (U) resp. du mélange de gaz de comparaison provenant de la seconde source (112) par une addition d'au moins un gaz inerte de poids spécifique plus faible (I1) et d'au moins un gaz inerte de poids spécifique plus élevé (I2) et
(b) au moins un dispositif (12) pour déterminer les sommes des masses moléculaires (MM) des mélanges de gaz exhalés par le patient, l'au moins un gaz inerte de poids spécifique plus élevé (I2) dans le mélange de gaz de test étant choisi dans le groupe comprenant l'argon, le néon, le krypton, le radon, le xénon et le SF₆ ainsi que des mélanges de ces gaz, le radon et le SF₆ étant préférés et le SF₆ étant particulièrement préféré.

2. Dispositif (10) selon la revendication 1, **caractérisé par le fait que** la somme de masse moléculaire du mélange de gaz de test présente avec la somme de masse moléculaire de l'air ambiant (U) ou du mélange de gaz de comparaison provenant d'une seconde source (112) une différence de moins de 10%, de préférence moins de 5%, plus préférablement moins de 2%, idéalement moins de 1 %.

3. Dispositif (10) selon la revendication 1 ou 2, **caractérisé par le fait que** le mélange de gaz de comparaison provenant de la seconde source (112) contient de l'oxygène en concentration de 21 à 100, de préférence de 30 à 100, plus préférablement de 50 à 100, idéalement de 100% en volume.

4. Dispositif (10) selon l'une des revendications 1 à 3, **caractérisé par le fait que** le mélange de gaz de test présente une somme de masse moléculaire de 28 à 33 g/mol, une somme de masse moléculaire d'environ 29 ou 32, notamment d'environ 28,85 ou 32,0 g/mol, étant préférée.

5. Dispositif (10) selon l'une des revendications 1 à 4, **caractérisé par le fait que** l'au moins un gaz inerte de poids spécifique plus faible dans le mélange de gaz de test est l'hélium.

6. Dispositif (10) selon l'une des revendications 1 à 5, **caractérisé par le fait que** le dispositif (12) pour déterminer la somme de masse moléculaire (MM) du mélange de gaz de test exhalé par le patient (P) comprend un appareil de mesure de la vitesse des ultrasons dans les gaz ou les mélanges de gaz et un dispositif (121) de conversion des vitesses des ultrasons mesurées en la somme de masse moléculaire, ainsi qu'un dispositif (122) d'affichage des valeurs mesurées.

7. Dispositif (10) selon l'une des revendications 1 à 6, **caractérisé par le fait qu'**il comprend un mélange de gaz de comparaison qui présente essentiellement la même somme de masse moléculaire que l'air ambiant, avec toutefois une plus grande proportion d'oxygène que celui-ci, et auquel le gaz à poids spécifique plus élevé n'est pas ajouté.

8. Dispositif (10) selon l'une des revendications 1 à 7, **caractérisé par le fait que** le dispositif (12) pour la détermination de la somme de masse moléculaire comprend un dispositif (123) pour convertir les valeurs mesurées en conditions normales.

9. Dispositif (10) selon l'une des revendications 1 à 8, **caractérisé par le fait qu'**il comprend les dispositifs (14; 140; 141; 142) pour passer du mélange de gaz inhalé par le patient (P) provenant de l'air ambiant (U) au mélange de gaz de test délivré par la source (11) et inversement, resp. pour passer du mélange de gaz de test inhalé par le patient (P) provenant de la source (112) au mélange de gaz de test provenant de la source (11) et inversement.

10. Mélange de gaz de test pour déterminer l'homogénéité de la ventilation (VH) chez un patient respirant spontanément ou ventilé, **caractérisé par le fait que** le mélange de gaz de test est un mélange de gaz respirable qui présente essentiellement la même somme de masse moléculaire (MM) que l'air ambiant (U) ou qu'un mélange de gaz de comparaison, mais qui s'en distingue par une addition d'au moins un gaz inerte de poids spécifique plus élevé (I2), l'au moins un gaz inerte de poids spécifique plus élevé (I2) dans le mélange de gaz de test étant choisi dans le groupe comprenant l'argon, le néon, le krypton, le radon, le xénon et le SF₆ ainsi que des mélanges de ces gaz, le radon et le SF₆ étant préférés et le SF₆ étant particulièrement préféré.

11. Mélange de gaz de test selon la revendication 10, **caractérisé par le fait que** la somme de masse moléculaire du mélange de gaz de test présente avec la somme de masse moléculaire de l'air ambiant (U) ou du mélange de gaz de comparaison une différence de moins de 10%, de préférence moins de 5%, plus préférablement moins de 2%, idéalement moins de 1%.

12. Mélange de gaz de test selon la revendication 10 ou 11, **caractérisé par le fait que** le mélange de gaz de test présente une somme de masse moléculaire de 28 à 33 g/mol, une somme de masse moléculaire d'environ 29 ou 32, notamment d'environ 28,85 ou 32,0 g/mol, étant préférée.

13. Mélange de gaz de test selon l'une des revendications 10 à 12, **caractérisé par le fait que** l'au moins un gaz inerte de poids spécifique plus faible est l'hélium.

14. Utilisation d'un mélange de gaz de test selon l'une des revendications 10 à 13 dans un dispositif selon l'une des revendications 1 à 9.

15. Procédé de détermination de l'homogénéité de la ventilation (VH) chez un patient (P) respirant spontanément ou ventilé, **caractérisé par le fait que** le patient:
inhale un mélange de gaz de test respirable de manière active ou de manière passive et contrôlée, mélange qui présente essentiellement la même somme de masse moléculaire (MM) que l'air ambiant ou un mélange de gaz de comparaison mais qui se distingue des deux par une addition d'au moins un gaz inerte de poids spécifique plus élevé (I2) et une proportion suffisante d'un gaz inerte de poids spécifique plus faible (I1) pour faire en sorte que la somme de masse moléculaire du gaz de test ait pratiquement la même valeur que la somme de masse moléculaire de l'air ambiant ou du gaz de comparaison, et **par le fait que** la somme de masse moléculaire (MM) du gaz de test exhalé par le patient est déterminée afin d'évaluer l'homogénéité de la ventilation du patient à partir des différences entre les sommes des masses moléculaires (MM) du mélange de gaz de test inhalé et exhalé par le patient.
